# EUROPÄISCHE PATENTANMELDUNG

(11) **EP 0 557 587 A1**
(43) Veröffentlichungstag der Anmeldung: **01.09.1993**
(21) Anmeldenummer: 92120012.7
(22) Anmeldetag: 25.11.1992
(51) Int. Cl.: G02B 6/10, G02B 6/42

(54) **Bauteil für die Übertragung von energiereichem Licht und Verwendung des Bauteils**

(30) Priorität: 28.02.1992 DE 4206182
(71) Anmelder: Heraeus Quarzglas GmbH, D-63405 Hanau (DE)
(72) Erfinder: Fabian, Heinz, W-6450 Hanau 7 (DE); Thomas, Stephan, Dr., W-6451 Grosskrotzenburg (DE)
(74) Vertreter: Kühn, Hans-Christian

(57) **Zusammenfassung**

Es wird ein Bauteil für die Übertragung von Licht hoher Energiedichte mit einer Wellenlänge zwischen 250 nm und 400 nm aus synthetischem, hochreinem Quarzglas beschrieben, bei dem zur Erzielung einer möglichst geringen Transmissionsänderung bei der Übertragung des Lichtes, das Quarzglas einen Hydroxylionengehalt im Bereich zwischen 50 ppm und 1200 ppm und einen unterstöchiometrischen Gehalt an Sauerstoff aufweist.

## Beschreibung

Die Erfindung betrifft ein Bauteil für die Übertragung von Licht hoher Energiedichte mit einer Wellenlänge zwischen 250 nm und 400 nm, aus synthetischem, hochreinem Quarzglas, mit einem, eine Lichteintrittsfläche aufweisenden Lichteinkoppelbereich, einem, eine Lichtaustrittsfläche aufweisenden Lichtauskoppelbereich und einer zwischen Lichteinkoppelbereich und Lichtauskoppelbereich angeordneten Lichtübertragungsstrecke und die Verwendung des Bauteils.

Derartige Bauteile werden zur Übertragung von ultraviolettem Licht mit großer Energiedichte, insbesondere für die Übertragung des Lichtes von Excimer-Lasern, beispielsweise zur Materialbearbeitung oder im medizinischen Bereich zur Behandlung von Gefäßerkrankungen oder in der Ophthalmologie, eingesetzt. Die Anwendung derartiger Bauteile für die Übertragung von Laserstrahlung im ultravioletten Spektralbereich wird jedoch durch die sogenannte "Photodegradation" eingeschränkt. Darunter wird die Abnahme der Transmission aufgrund der durch die energiereiche Strahlung induzierten Dämpfung verstanden. Dieser Effekt der "Photodegradation", der umso ausgeprägter ist, je größer die Energiedichte der zu übertragenen Lichtstrahlung ist, wurde unter anderem auch für die Excimer-Laserwellenlängen von 351 nm (XeF), 308 nm (XeCl) und 248 nm (KrF) beobachtet. Neben deutlich sichtbaren makroskopischen Defekten, wie Aufschmelzungen der Oberfläche, Abplatzungen oder Rissen, kann die Abnahme der Transmission durch mikroskopische Störungen der Glasstruktur hervorgerufen werden.

Aus der EP-A2 0 401 845 sind optische Bauteile zur Übertragung von ultraviolettem Licht mit einer Wellenlänge, die kürzer als 360 nm ist bekannt, wobei die Bauteile aus hochreinem, schlierenfreiem, synthetischem Quarzglas bestehen, das Hydroxylionen mit einem Gehalt von mindestens 50 ppm enthält und das mit Wasserstoff dotiert ist. Es wird darin erläutert, daß der Wasserstoff und der Hydroxylionengehalt des Glases einen positiven Einfluß auf die Beständigkeit der Gläser gegenüber gepulsten Excimer-Laserstrahlen haben können. Gegenüber Bauteilen aus reinem Quarzglas zeichnen sich die bekannten Bauteile durch eine verbesserte Strahlenbeständigkeit aus. Insbesondere sind mittels Bauteilen aus derartigen Quarzgläsern höhere Energiedichten im Ultravioletten übertragbar, bevor eine merkliche Materialschädigung zu beobachten ist. Das heißt, die Photodegradations-Effekte der Wasserstoff- und Hydroxylionenhaltigen Quarzgläser treten gegenüber denjenigen von reinem Quarzglas erst bei höheren Energiedichten auf. Weiterhin ist aus dieser Schrift bekannt, daß metallische Verunreinigungen die Beständigkeit des Quarzglases gegen Laserstrahlen mit hohem Energiegehalt herabsetzen können.

Im Aufsatz von Rod S. Taylor et al., "Dependence of the damage and transmission properties of fused silica fibers on the excimer laser wavelength", veröffentlicht in APPLIED OPTICS, Vol. 27, No. 15 (1988) wird die Strahlenbeständigkeit von Bauteilen im Hinblick auf die Übertragung definierter Excimer-Laserstrahlung im Wellenlängenbereich von 193 nm bis 351 nm untersucht. Bei den untersuchten Bauteilen handelt es sich um Fasern aus undotiertem, synthetischem Quarzglas mit einem Gehalt an Hydroxylionen zwischen 325 ppm und 1200 ppm. Es wird gezeigt, daß mit Ausnahme der 351 nm-Excimer-Laserwellenlänge, die Transmission der Fasern mit zunehmender Betriebsdauer abnimmt, wobei jedoch einige Zeit nach der Bestrahlung mit dem energiereichen Laserlicht eine teilweise Erholung der Transmission zu beobachten ist. Weiterhin wurde in diesem Aufsatz in einem direkten Vergleich der Transmissionsänderung von Quarzglas-Fasern mit einem Hydroxylionengehalt von 400 ppm gegenüber solchen mit 1200 ppm gefunden, daß bei gleicher Wellenlänge und gleicher Energie der von den Fasern übertragenen Laserstrahlung, die Faser mit dem Hydroxylionengehalt von 400 ppm die geringere Transmissionsänderung erfährt.

Aus dem Artikel von C. Whitehurst, et al, "Ultraviolet pulse transmission in optical fibres", Journal of modern optics, 1988, vol. 35, No. 3, 371-385, sind Bauteile bekannt, die aus Quarzglas bestehen, das bis zu 1500 ppm Hydroxylionen enthält und das gegenüber reinem, "trockenem" Quarzglas eine zu höheren Energiedichten hin verschobene "Zerstörschwelle" aufweist. In diesem Artikel ist auch die Ausbildung von Bauteilen aus hydroxylionenhaltigem Quarzglas mit einem, eine Lichteintrittsfläche aufweisenden Lichteinkoppelbereich, einem, eine Lichtaustrittsfläche aufweisenden Lichtauskoppelbereich und einer zwischen Lichteinkoppelbereich und Lichtauskoppelbereich angeordneten Lichtübertragungsstrecke beschrieben, bei denen der Lichteinkoppelbereich in Form eines in Einstrahlrichtung sich verjüngenden Konus ausgebildet ist.

Anhand des Aufsatzes "Defects and Stress Phenomena in Optical Fibers" von H. Nishikawa et al., Proc OFC 1989, Paper THIl, sind Messungen des Absorptionsverhaltens optischer Fasern bekannt geworden, wobei die Fasern durch ihren Gehalt an Sauerstoffionen, das Verfahren ihrer Herstellung, ihren Chlorionengehalt und ihren Hydroxylionengehalt charakterisiert werden. An den Fasern mit unterstöchiometrischem Gehalt an Sauerstoff konnte eine Absorptionsbande bei 245 nm gemessen werden, wobei diese Absorptionsbande einer, aufgrund des unterstöchiometrischen Sauerstoffgehaltes, intrinsisch vorhandenen Defektart der Glasstruktur, nämlich sogenannten "Sauerstofflücken" zugeordnet wird.

Ähnliche Messungen wurden auch von R. Thomon et al. in dem Aufsatz "Correlation of the 5.0- and 7.6-eV absorption bands in SiO₂ with oxygen vacancy", PHYSICAL REVIEW B, Vol. 39 (1989), No. 2, veröffentlicht. Aufgrund von Messungen und entsprechenden Berechnungen wird dargelegt, daß die bei verschiedenen, hochreinen Quarzgläsern beobachteten Absorptionsbanden bei 5.0 eV (245 nm) auf Sauerstofflücken und andere Sauerstoffdefizit-Defekte zurückzuführen sind. Die für die Messungen verwendeten Quarzgläser werden dabei ebenfalls durch die Art ihrer Herstellung sowie durch ihre Gehalte an Chlorionen und Hydroxylionen charakterisiert.

Gegenüber energiereicher Strahlung im Wellenlängenbereich zwischen 250 nm und 400 nm zeigen die bekannten Bauteile aus Quarzglas eine Abnahme der Transmission von Beginn der Bestrahlung an. Ausgehend von einem Anfangswert nimmt dabei die Transmission der Bauteile normalerweise bis zu einem "Plateauwert" ab, ab dem sie sich auch bei längerer Bestrahlung nur noch wenig verändert. Es hat sich gezeigt, daß diese Änderungen der Transmission von ihrem Anfangswert bis zu dem "Plateauwert" mit wachsender Energiedichte der zu übertragenden Strahlung deutlich ansteigen. Für zahlreiche Anwendungen sind jedoch größere Transmissionsänderungen im Verlaufe des Einsatzes des Bauteils nicht tolerierbar. Da aber andererseits gerade die maximal transmittierbare Energiedichte für die meisten Anwendungen der entscheidende Parameter ist, wird der Einsatzbereich der bekannten Bauteile durch den Effekt dieser "Photodegradation" stark eingeschränkt.

Der vorliegenden Erfindung liegt die Aufgabe zugrunde, ein Bauteil anzugeben, das eine möglichst geringe Transmissionsänderung bei der Übertragung von Licht hoher Energiedichte und einer Wellenlänge zwischen 250 nm und 400 nm aufweist.

Diese Aufgabe wird erfindungsgemäß dadurch gelöst, daß, ausgehend von dem bekannten Bauteil, das Quarzglas einen Hydroxylionengehalt im Bereich zwischen 50 ppm und 1200 ppm und einen unterstöchiometrischen Gehalt an Sauerstoff aufweist. Durch die Übertragung ultravioletter Strahlung werden im Material des Bauteils zwangsläufig Defekte erzeugt. Die Art der Defekte, deren Konzentration sowie deren Auswirkungen auf die Transmission hängen im wesentlichen von der Energiedichte und der Wellenlänge der zu übertragenden Strahlung ab. Dabei können unter anderem sogenannte "Sauerstoffüberschußdefekte" entstehen, die zur Bildung oder Verstärkung von Absorptionsbanden bei verschiedenen Wellenlängen im UV-und IR-Spektrum führen können. Insbesondere hat es sich gezeigt, daß eine Absorptionsbande mit einem Maximum bei 265 nm mit der Bildung derartiger "Sauerstoffüberschußdefekte" korrelierbar ist. Neben der Charakteristik des zu übertragenden Lichtes hängt die Bildungswahrscheinlichkeit für derartige Defekte auch von der Glasstruktur, insbesondere von der Stärke und der Art der atomaren Bindungen sowie von der Koordination der Siliciumatome bzw. der mittleren Häufigkeit der Sauerstoffionen in der Nachbarschaft des Siliciums ab.

Durch den unterstöchiometrischen Gehalt an Sauerstoff im Quarzglas wird gewährleistet, daß die Wahrscheinlichkeit der Ausbildung derartiger "Sauerstoffüberschußdefekte" bei der Übertragung von energiereicher Strahlung mit einer Wellenlänge zwischen 250 nm und 400 nm verringert wird.

Andererseits würde jedoch auch eine allmähliche Anhäufung derartiger Defekte zu einer sich stetig verschlechternden Transmission des Bauteils führen Durch den Hydroxylionengehalt des Quarzglases kann aber bekanntermaßen ein Ausheilen bereits entstandener Defekte erreicht werden. Im Verlaufe der Übertragung von energiereichem Licht bildet sich dann durch die miteinander konkurrierenden Effekte der Defekt-Bildung und der Defekt-Ausheilung ein annäherndes Defekt-Gleichgewicht im Bauteil aus, das den "Plateauwert" der Transmission; also deren Abnahme vom ursprünglichen Transmissionswert, bestimmt. Es hat sich nun überraschend gezeigt, daß die bei der Übertragung von ultraviolettem, energiereichem Licht mit einer Wellenlänge zwischen 250 nm bis 400 nm in "sauerstoffarmem", hochreinem Quarzglas erzeugten Defekte derart sind, daß die im Quarzglas-Netzwerk vorhandenen Hydroxylionen, insbesondere der darin enthaltene Wasserstoff, eine besonders effektive Ausheilung dieser Defekte bewirken können, so daß die Abnahme der Transmission vom ursprünglichen Wert bis auf den "Plateauwert" sehr gering ist.

Als "hochrein" wird dabei ein Quarzglas bezeichnet, dessen Gehalt an Alkaliionen insgesamt weniger als 150 ppb, an Erdalkaliionen insgesamt weniger als 100 ppb und an anderen metallischen Verunreinigungen wie Ti, Cr, Fe, und Ni insgesamt weniger als 50 ppb beträgt.

Als besonders vorteilhaft hat sich ein Bauteil erwiesen, bei dem das Quarzglas einen Hydroxylionengehalt von weniger als 600 ppm aufweist und der unterstöchiometrische Gehalt an Sauerstoff derart ist, daß das Quarzglas eine Absorptionsbande mit einem Maximum im Wellenlängenbereich von 240 nm bis 250 nm mit einer Intensität von mehr als 0,1 dB/m aufweist. Als Intensität der Absorptionsbande ist hierbei der Dämpfungswert in dB/m gemeint, der der Höhe des Maximums der Absorptionsbande über der Grundlinie der Absorptionsbande entspricht. Dadurch, daß der unterstöchiometrische Gehalt an Sauerstoff mindestens so groß ist, daß er im Quarzglas eine Absorptionsbande mit einer Intensität von mindestens 0,1 dB/m im Maximum erzeugt, wird gewährleistet, daß so wenig "Sauerstoffüberschußdefekte" entstehen, daß die durch sie erzeugbare Absorption bei 265 nm nicht oder kaum merklich auftritt und daß gleichzeitig die durch die energiereiche Strahlung mit Wellenlängen zwischen 250 nm und 400 nm in dem derartig sauerstoffarmen Quarzglas erzeugten Defekte derart sind, daß sie entweder von relativ geringen Hydroxylionenkonzentrationen von 50 ppm bis 600 ppm sehr effektiv ausgeheilt werden können oder zumindest im Wellenlängenbereich um 250 nm bis ca. 400 nm keine oder wenig Absorption erzeugen und daher die Übertragung energiereicher Strahlung in diesem Wellenlängenbereich nicht oder kaum behindern. Dabei wirkt sich der relativ geringe Hydroxylionengehalt des Quarzglases von weniger als 600 ppm auf das Transmissionsverhalten des Quarzglases vorteilhaft aus. Es hat sich nämlich gezeigt, daß hohe Hydroxylionengehalte in Quarzgläsern ebenfalls zu einer Defektbildung bei der Übertragung energiereicher Strahlung beitragen können. Ein Hydroxylionengehalt von mindestens 200 ppm hat sich aber als günstig erwiesen.

Insbesondere hinsichtlich einer möglichst ungestörten Übertragung von Licht hoher Energiedichte über einen großen Wellenlängenbereich hat es sich als vorteilhaft erwiesen, daß das Bauteil Quarzglas aufweist, das im Wellenlängenbereich zwischen 200 nm und 350 nm nur eine einzige Absorptionsbande aufweist.

Für die Übertragung von energiereichem Licht über eine längere Strecke hat es sich bewährt, den Lichtübertragungsbereich in Form einer Faser oder in Form eines Stabes auszubilden. Dabei ist es besonders günstig, den Lichteinkoppelbereich und/oder den Lichtauskoppelbereich in Form eines in Richtung auf den Lichtübertragungsbereich sich verjüngenden Konus auszubilden. Da die Zerstörschwelle der Oberfläche eines Bauteils gegenüber derjenigen des Vollmaterials herabgesetzt ist, können bei der Einkopplung bzw. auch bei der Auskopplung von Strahlung bereits bei Leistungsdichten, die im Vollmaterial des Bauteils selbst übertragen werden könnten, Schädigungen oder Zerstörungen der Einkoppel- bzw. der Auskoppelfläche auftreten. Durch eine Vergrößerung dieser Einkoppel- bzw. Auskoppelflächen gegenüber dem Querschnitt der eigentlichen Lichtübertragungsstrecke und eine damit einhergehende mögliche Aufweitung des einzukoppelnden bzw. des auszukoppelnden Lichtstrahles, wird die optische Belastung pro Flächeneinheit der jeweiligen Fläche verringert.

Dies ermöglicht die Übertragung energiereicher Strahlung, die andernfalls aufgrund ihrer hohen optischen Leistungsdichten die Oberflächen des Bauteils beim Ein- bzw. beim Auskoppeln zerstören würden.

Zur Kompensation spezieller Strahlcharakteristiken des einzukoppelnden bzw. des auszukoppelnden Lichtes hat es sich auch als vorteilhaft erwiesen, die Lichteintrittsfläche und/oder die Lichtaustrittsfläche gekrümmt auszubilden.

Insbesondere für die Führung von energiereicher Strahlung über längere Strekken oder Biegungen hat es sich als günstig erwiesen, mindestens die Lichtübertragungsstrecke des Bauteils mit einem Mantelmaterial zu umhüllen, dessen Brechungsindex kleiner als 1,4589 ist. Als Mantelmaterial haben sich insbesondere Fluor und/oder Bor dotiertes Quarzglas oder gegen ultraviolette Strahlen beständiger Kunststoff bewährt.

Als besonders vorteilhaft hat sich der Einsatz des erfindungsgemäßen Bauteils für die Übertragung von energiereichem Licht im Wellenlängenbereich zwischen 300 nm und 320 nm, insbesondere mit einer Wellenlänge um 310 nm erwiesen. Die aufgrund der Strahlung in diesem Wellenlängenbereich erzeugten Defekte werden entweder besonders leicht in dem erfindungsgemäßen Bauteil ausgeheilt oder sie erzeugen lediglich solche Absorptionsbanden, die für die Übertragung von Licht in diesem Wellenlängenbereich nicht störend sind.

In einer Ausführungsform, bei der der Lichtübertragungsbereich in Form einer Faser oder in Form eines Stabes ausgebildet ist, hat sich das erfindungsgemäße Bauteil auch zur Verwendung als Einzelelement in einer flexiblen Anordnung mehrerer, mit ihren Längsachsen parallel oder im wesentlichen parallel zueinander verlaufender, gleicher oder geometrisch ähnlicher Einzelelemente für die Übertragung von ultraviolettem Licht mit hohen Energiedichten zur Materialbehandlung bewährt. Dabei können die Bauteile beispielsweise in einem aushärtbaren Material eingebettet und/oder gegeneinander verdrillt sein.

Für spezielle Anwendungen hat sich ein Bauteil bewährt, bei dem der Lichtübertragungsbereich in Form eines dickwandigen Hohlzylinders oder in Form einer Lochscheibe ausgebildet ist. Diese Ausführungsform des erfindungsgemäßen Bauteils ist insbesondere bei der Übertragung von ultraviolettem Licht als Strahlaufweiter und/oder zur Homogenisierung der von einzelnen Lichtstrahlen eines Strahlerbündels ausgehenden Lichtenergien in einer Ebene senkrecht zur Licht-Ausbreitungsrichtung geeignet. Es hat sich gezeigt, daß energiereiche Strahlung aus dem Wellenlängenbereich zwischen 250 nm und 400 nm, die beispielsweise von einzelnen, zu einem Bündel zusammengefaßten Lichtwellenleitern ausgeht, vorteilhaft in ein derartiges Bauteil eingekoppelt werden kann, wobei die von den einzelnen Lichtwellenleitern ausgehenden Lichtmoden sich im Verlaufe ihrer Übertragung über die Länge der Lichtübertragungsstrecke des Bauteils miteinander vermischen. Hierzu wird die Länge der Lichtübertragungsstrecke vorteilhafterweise so gewählt, daß an der dem Lichtwellenleiter-Bündel abgewandten Seite des Bauteils, die Lichtenergien der einzelnen Lichtstrahlen aufgrund ihrer Divergenz zumindest teilweise überlappen. Ein zu behandelndes oder abzutragendes Material oder Gewebe kann dadurch gleichmäßig über eine größere Fläche mit hoher Energie beaufschlagt werden. Der Kanal in dem Hohlzylinder kann dabei der Zufuhr von gasförmigen oder flüssigen Medien sowie zur Durchführung von Führungsdrähten dienen. Dies ist beispielsweise bei der ablativen Behandlung von Ablagerungen in Blutgefäßen mittels eines medizinischen Katheters, der aus einzelnen, mit ihren Längsachsen parallel zueinander verlaufenden Einzelfasern aufgebaut ist, über welche das Arbeitslicht übertragen wird, von Bedeutung.

Ein Ausführungsbeispiel der Erfindung ist in der Zeichnung dargestellt und wird nachstehend näher erläutert. In der Zeichnung zeigt
- Figur 1: eine Messung der Dämpfung von verschiedenen Lichtwellenleitern und
- Figur 2: eine Messung des Photodegradations-Verhaltens eines erfindungsgemäßen Bauteils bei der Übertragung von Licht hoher Energiedichte mit hohen Pulszahlen, im Vergleich zu dem Photodegradations-Verhaltens eines Bauteils gemäß dem Stand der Technik.

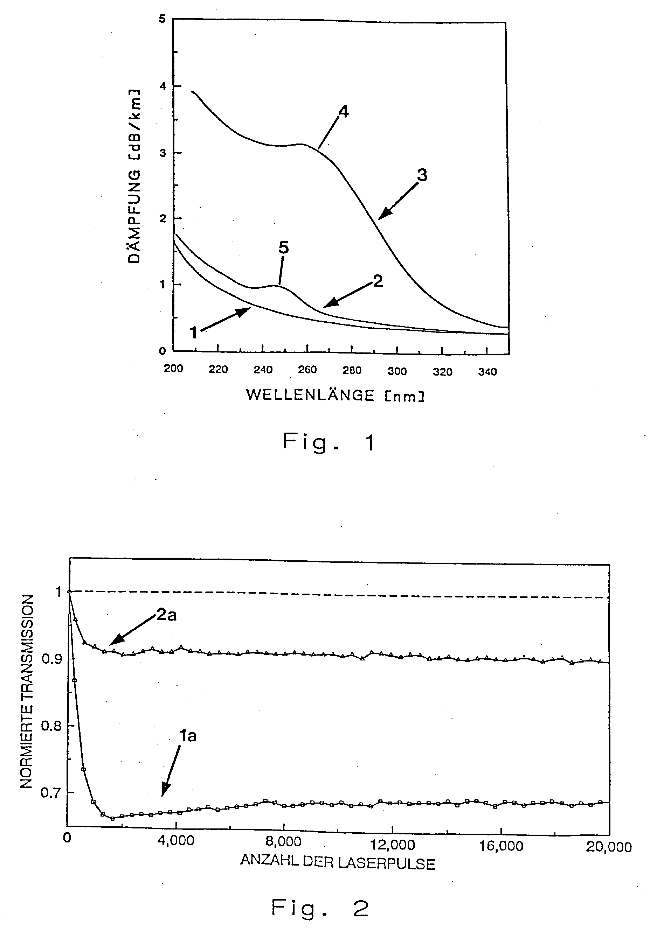

Die in der Figur 1 dargestellten Kurven 1, 2 und 3 stellen den Verlauf der Dämpfung von Lichtwellenleitern dar, die nach jeweils dem gleichen Verfahren aus undotiertem, hochreinem, synthetischem Quarzglas hergestellt worden sind. Das Material der Lichtwellenleiter unterscheidet sich lediglich aufgrund des unterschiedlichen Sauerstoffangebotes während der Materialherstellung. Die Lichtwellenleiter 1, 2, 3 wurden aus Vorformen gezogen, deren Hydroxylionengehalt jeweils 600 ppm betrug.

Das Quarzglas für den Lichtwellenleiter 1 wurde mit einem Sauerstoffangebot hergestellt, das der Menge entspricht, die zur Herstellung eines Quarzglases mit korrekter Materialstöchiometrie (Silicium : Sauerstoff = 1 : 2) notwendig ist. Bei der Herstellung des Materials für den Lichtwellenleiter 2 wurde das Sauerstoffangebot im Vergleich dazu deutlich verringert, während bei der Herstellung des Quarzglases für den Lichtwellenleiter 3 ein Sauerstoffüberangebot gewählt wurde.

Mit Hilfe eines Plasma-Außenbeschichtungsverfahrens wurden auf die so hergestellten Quarzglaszylinder fluordotierte Quarzglas-Mantelschichten aufgebracht, wobei das Verhältnis zwischen dem Außendurchmesser des Mantels und dem Kerndurchmesser 1,1 betrug. Aus den so hergestellten Vorformen wurden dann die Lichtwellenleiter 1, 2 und 3 mit jeweils einem Faserdurchmesser von 220 µm gezogen.

Der Verlauf der Grunddämpfung der Lichtwellenleiter 1, 2, 3 im Spektralbereich zwischen 200 nm und 350 nm weist signifikante Unterschiede auf, die allein auf das unterschiedliche Sauerstoffangebot bei der Quarzglasherstellung zurückzuführen sind. Der Lichtwellenleiter 1 zeigt einen Kurvenverlauf, der durch den Anstieg der Streuung zu kürzeren Wellenlängen hin bestimmt wird. Der Lichtwellenleiter 3, dessen Kern aus einem Quarzglas mit überstöchiometrischem Sauerstoffgehalt besteht, weist eine breite Absorptionsbande 4 mit einem Maximum bei ca. 265 nm auf, die die Kurvencharakteristik im untersuchten Spektralbereich bestimmt. Aus dieser Absorptionsbande 4 kann auf das Vorhandensein von sog. "Sauerstoffüberschußdefekten" geschlossen werden.

Die Grunddämpfung des Lichtwellenleiters 2, dessen Kern aus einem Quarzglas mit unterstöchiometrischem Sauerstoffgehalt besteht, weist dagegen eine relativ schwach ausgeprägte, schmalbandige Absorption S mit einem Maximum bei ca. 245 nm auf. Diese Bande 5, deren Intensität ca. 0,2 dB/m beträgt, läßt auf das Vorhandensein sog. "Sauerstofflücken" oder andere Sauerstoff-Defekte schließen.

Durch Teilstücke der Lichtwellenleiter 1 und 2 wurde anschießend energiereiches XeClExcimer-Laserlicht übertragen und das Photodegradations-Verhalten gemessen. Die Wellenlänge des Excimer-Laserlichtes betrug dabei 308 nm, die Pulsdauer 28 ns, die Energiedichte 15 J/cm² und die Impulsfrequenz 30 Hz. Bei den gemessenen Lichtwellenleitern 1a; 2a (Figur 2) handelte es sich um 2 m lange Probenstücke. Die Ergebnisse dieser Messungen sind in der Figur 2 dargestellt und zeigen den starken Einfluß des Sauerstoffangebotes bei der Kernmaterialherstellung auf die Photodegradationseigenschaften der daraus hergestellten Lichtwellenleiter.

Der mit der Bezugsziffer 1a bezeichnete Lichtwellenleiter, der dem Stand der Technik entspricht, zeigt einen raschen Abfall der ursprünglichen Transmission auf einen "Plateauwert" und nach ca. 20.000 Laserpulsen eine Transmission von ca. 70 % gegenüber dem Wert bei Bestrahlungsbeginn. Der mit der Bezugsziffer 2a bezeichnete Lichtwellenleiter dessen Kern aus sauerstoffarmem Quarzglas besteht, zeigt hingegen ein besseres Photodegradationsverhalten. Nach 20.000 Laserpulsen liegt die Transmission bei diesem Lichtwellenleiter 2a noch oberhalb von 90 % ihres ursprünglichen Wertes. Die Transmission des Lichtwellenleiters 2a betrug selbst nach 100.000 Laserpulsen immer noch 90 % des ursprünglichen Wertes.

## Patentansprüche

1. Bauteil für die Übertragung von Licht hoher Energiedichte mit einer Wellenlänge zwischen 250 nm und 400 nm, aus synthetischem, hochreinem Quarzglas, mit einem, eine Lichteintrittsfläche aufweisenden Lichteinkoppelbereich, einem, eine Lichtaustrittsfläche aufweisenden Lichtauskoppelbereich und einer zwischen Lichteinkoppelbereich und Lichtauskoppelbereich angeordneten Lichtübertragungsstrecke, dadurch gekennzeichnet, daß das Quarzglas einen Hydroxylionengehalt im Bereich zwischen 50 ppm und 1200 ppm und einen unterstöchiometrischen Gehalt an Sauerstoff aufweist.

2. Bauteil nach Anspruch 1, dadurch gekennzeichnet, daß das Quarzglas einen Hydroxylionengehalt von weniger als 600 ppm aufweist und der unterstöchiometrische Gehalt an Sauerstoff derart ist, daß das Quarzglas eine Absorptionsbande (5) mit einem Maximum im Wellenlängenbereich von 240 nm bis 250 nm mit einer Intensität von mehr als 0,1 dB/m aufweist.

3. Bauteil nach Anspruch 1 oder 2, dadurch gekennzeichnet, daß das Quarzglas einen Hydroxylionengehalt von mindestens 200 ppm aufweist.

4. Bauteil nach einem der Ansprüche 1 bis 3, dadurch gekennzeichnet, daß das Quarzglas im Wellenlängenbereich zwischen 200 nm und 350 nm nur eine einzige Absorptionsbande (5) aufweist.

5. Bauteil nach einem der Ansprüche 1 bis 4, dadurch gekennzeichnet, daß der Lichtübertragungsbereich in Form einer Faser oder in Form eines Stabes ausgebildet ist.

6. Bauteil nach Anspruch 5, dadurch gekennzeichnet, daß der Lichteinkoppelbereich und/oder der Lichtauskoppelbereich in Form eines sich in Richtung auf die Lichtübertragungsstrecke verjüngenden Konus ausgebildet ist.

7. Bauteil nach einem oder mehreren der Ansprüche 1 bis 6, dadurch gekennzeichnet, daß die Lichteintrittsfläche und/oder die Lichtaustrittsfläche gekrümmt ist.

8. Bauteil nach einem oder mehreren der Ansprüche 1 bis 7, dadurch gekennzeichnet, daß mindestens die Lichtübertragungsstrecke von einem Mantelmaterial umhüllt ist, das einen Brechungsindex von weniger als 1,4589 aufweist.

9. Bauteil nach Anspruch 8, dadurch gekennzeichnet, daß das Mantelmaterial mit Fluor und/oder Bor dotiertes Quarzglas enthält.

10. Bauteil nach Anspruch 8, dadurch gekennzeichnet, daß das Mantelmaterial einen gegen ultraviolette Strahlen beständigen Kunststoff enthält.

11. Bauteil nach einem oder mehreren der Ansprüche 1 bis 4 oder 6 bis 10, dadurch gekennzeichnet, daß die Lichtübertragungsstrecke in Form eines dickwandigen Hohlzylinders oder einer Lochscheibe ausgebildet ist.

12. Verwendung eines Bauteils nach einem oder mehreren der Ansprüche 1 bis 11 für die Übertragung von Licht hoher Energiedichte mit einer Wellenlänge im Bereich zwischen 300 nm und 320 nm, insbesondere mit einer Wellenlänge von 308 nm.

13. Verwendung eines Bauteils nach Anspruch 5 und einem oder mehreren der Ansprüche 1 bis 4 und 6 bis 10 als Einzelelement in einer flexiblen Anordnung mehrerer, mit ihren Längsachsen im wesentlichen parallel zueinander verlaufender, gleicher oder geometrisch ähnlicher Einzelelemente für die Übertragung von ultraviolettem Licht mit hohen Energiedichten zur Materialbehandlung.

14. Verwendung eines Bauteils nach Anspruch 11 bei der Übertragung von ultraviolettem Licht als Strahlaufweiter und/oder zur Homogenisierung der von einzelnen Lichtstrahlen eines Strahlerbündels ausgehenden Lichtenergien in einer Ebene senkrecht zur Licht-Ausbreitungsrichtung.
